# EUROPÄISCHE PATENTSCHRIFT

(11) **EP 4 326 205 B1**
(45) Veröffentlichungstag und Bekanntmachung des Hinweises auf die Patenterteilung: **17.06.2026**
(21) Anmeldenummer: 22722172.8
(22) Anmeldetag: 08.04.2022
(51) Int. Cl.: A61F 13/15, A61F 13/20, B29C 65/18

(54) **VERFAHREN ZUM WICKELN VON VORFORMLINGEN**
METHOD FOR WINDING PREFORMS
PROCÉDÉ D'ENROULEMENT DE PRÉFORMES

(30) Priorität: 21.04.2021 CH 4262021
(43) Veröffentlichungstag der Anmeldung: 28.02.2024
(73) Patentinhaber: Ruggli AG, 5322 Koblenz (CH)
(72) Erfinder: SCHULER, Samuel, 4051 Basel (CH); AUER, Marco, 5325 Leibstadt (CH)
(74) Vertreter: IPrime Rentsch Kaelin AG
(86) Internationale Anmeldenummer: PCT/EP2022/059526
(87) Internationale Veröffentlichungsnummer: WO 2022/223330

(56) Entgegenhaltungen:
- CH-A1- 714 846

## Beschreibung

Die vorliegende Erfindung betrifft ein Verfahren zum Wickeln von Vorformlingen zur Herstellung von Tampons aus bandförmigem Material, sowie eine entsprechende Vorrichtung zum Wickeln von Vorformlingen, beides gemäss Oberbegriff der kennzeichnenden Ansprüche.

### Technologischer Hintergrund

Für die weibliche Hygiene, insbesondere während der Regelblutung verwendete Tampons bestehen im Wesentlichen aus einem saugfähigen Material, meistens einem Viskose-Wattestreifen, welches zur charakteristischen, länglichen Form zunächst gewickelt, dann verpresst und geformt wird. Die für die Herstellung der Tampons verwendeten Vorformlinge sind Wickel aus Streifen dieses bandförmigen Materials. Die Streifen haben in der Regel eine Länge von zwischen 150 und 350 mm und eine Breite von zwischen 30 und 60 mm. In der Regel sind die verwendeten Materialien in einer stoffschlüssigen Verbindung miteinander verbunden, wobei meistens ein thermisch induziertes Laminieren stattfindet, bei dem ein Schichtmaterial thermoplastisch mit der Watte verbunden wird. Das resultierende Laminatband wird in die geeignete Streifenlänge konfektioniert und mit einem Rückholfaden versehen, bevor es zu einem Wickel verwickelt wird. Der Rückholfaden wird in der Regel rechtwinkelig zur Längsachse des Laminatstreifens vor dem eigentlichen Wickelvorgang umgeschlagen.

In der Tamponproduktion spielen hohe Stückzahlen bei gleichzeitig hoher Qualitätsanforderung eine grosse Rolle. Beim Vorgang des Laminierens können erschwerend die unterschiedlichen Ausmasse des Wattebandes und des Vliesbandes hinzukommen. So sind bestimmte Tamponfertigungsverfahren darauf ausgelegt, einen endseitigen, proximalen Abschluss des Tampons zu erzeugen, indem ein überstehender Bereich des Vliesbandes um den Rückholfaden am distalen Ende thermoplastisch mit der Tamponoberfläche verbunden wird.

Die WO 2017/109166 A1 (Heege, T. et al.) beschreibt ein Verfahren, um kontinuierlich Streifen eines Laminats bereitzustellen, welche aus zwei Fasermaterialien bestehen. Dabei werden die Fasermaterialien mit unterschiedlichen Geschwindigkeiten einander angenähert und das zweite Fasermaterial, nach Zuschnitt auf die entsprechende Bandlänge, auf die Geschwindigkeit des ersten Bandmaterials bei gleichzeitigem thermoplastischem Verbinden beschleunigt.

Weitere Stand der Technik ist aus CH714846 bekannt.

Solche und ähnliche Herstellungsprozesse für Laminatstreifen sind bekannt. Die Herausforderung liegt darin, die Laminatstreifen der nachfolgenden Bearbeitungseinheit zuzuführen, ohne dass eine intermittierend operierende Übertragungseinheit dazwischenzuschalten ist. In gängigen Verfahren wird z.B. ein kontinuierlich erzeugter Laminatstreifen in einem Stop-and-go-Prozess auf eine Wickelnadel gebracht, welche den Laminatstreifen zum Vorformling verwickelt.

Somit besteht ein Bedarf nach Vorrichtungen und Verfahren zum Wickeln von Vorformlingen zur Herstellung von Tampons, welche im Anschluss zum Beispiel an eine kontinuierliche Laminierung und Konfektionierung von ein- oder mehrschichtigen Bändern eine ebenso kontinuierliche Weiterverarbeitung ermöglichen.

### Darstellung der Erfindung

Es ist somit eine Aufgabe der vorliegenden Erfindung, ein Verfahren und eine Vorrichtung zum Wickeln von Vorformlingen für die Tamponherstellung bereitzustellen, welches mindestens einen Nachteil des Bekannten überwindet. Insbesondere sollen ein entsprechendes Verfahren und eine dazugehörige Vorrichtung bereitgestellt werden, welche ein kontinuierliches Einspeisen konfektionierter Materialstreifen in eine kontinuierliche Tamponfertigung ermöglicht.

Diese Aufgabe wird durch die kennzeichnenden Merkmale der unabhängigen Ansprüche gelöst.

Ein Aspekt der vorliegenden Erfindung ist ein Verfahren zum Wickeln von Vorformlingen zur Herstellung von Tampons. Das erfindungsgemässe Verfahren umfasst eine Reihe von Schritten.

Zunächst wird ein Materialband zugeführt, insbesondere ein endloses Materialband. Anschliessend werden bandförmige Materialstreifen vom Materiaband vereinzelt.

Beim erfindungsgemässen Verfahren wird ein vereinzelter Materialstreifen mittels mindestens einer Antriebsrolle in eine kontinuierlich fördernde Wickeleinheit dergestalt eingeschoben, dass der Materialstreifen zwischen eine Wickelgabel und einem Schwert so positioniert wird, dass bei einer relativen Bewegung zwischen Wickelgabel und Schwert dieses einen Abschnitt des Materialstreifens in einen Gabelabstand drückt.

Durch das erfindungsgemässe Verfahren ist es möglich, eine kontinuierlich arbeitende Wickeleinheit direkt an eine kontinuierlich konfektionierende und wahlweise laminierende Zuführstation anzuschliessen, ohne dass dabei ein intermittierender Schritt erforderlich ist, bei dem z.B. eine der involvierten Werkstationen zur Übernahme des Werkstücks angehalten werden muss. Insbesondere ist eine kontaktfreie Übergabe zwischen einer Bandzuführstation und einer Wickelstation gewährleistet, zum Beispiel in dem kein Übernahmegreifer den Streifen von der Konfektionierung übernehmen, und in die Wickelstation überführen muss. Weiter wird durch das erfindungsgemässe Verfahren eine Tamponfertigung ermöglicht, welche Stückzahlen von über den bisher erreichten ermöglicht. Insbesondere werden Stückzahlen von über 140 Stück pro Minute erreicht, besonders bevorzugt Stückzahlen von um die 300 Stück pro Minute.

Im Sinne der vorliegenden Erfindung kann ein Materialband zum Beispiel als im Wesentlichen aus Fasermaterial bestehendes, oder Fasermaterial umfassendes Material verstanden werden.

Im Sinne der vorliegenden Erfindung kann als Fasermaterial ein natürliches oder synthetisches Material dienen, welches als Geflecht oder Gewebe von Fasern und Fäden gebildet ist. Herkömmlich für die Tamponproduktion sind Fasern aus einem Baumwollstoff oder einem Gemisch aus einem Baumwollstoff und einem Viskosefilament. Geeignet ist zum Beispiel ein Materialband aus hochreiner Verbandswatte aus 100% Viskose.

Das Materialband kann ein- oder mehrlagig ausgestaltet sein. Dabei können mehrfache Lagen aus demselben Material schichtweise vorliegen, oder verschiedene Materialien schichtweise vorliegen. Das Materialband kann auch nur teilweise aus mehreren Lagen ausgebaut sein, d.h. zum Beispiel in dem nur Teilabschnitte mehrlagig aufgebaut sind.

In einer besonderen Ausführungsform ist das Materialband ein Laminatband. In dieser Ausführungsform wird ein Trägermaterial mindestens einer ersten Zugrolle zugeführt. Bevorzugt handelt es sich beim Trägermaterial um ein Fasermaterial. Ein Schichtmaterial wird mittels mindestens einer zweiten Zugrolle ebenfalls zugeführt. Bevorzugt handelt es sich beim Schichtmaterial um einen thermoplastischen Vliesstoff. Das Trägermaterial und das Schichtmaterial werden zusammengeführt. Ein Laminatband wird gebildet, indem das Trägermaterial mit dem Schichtmaterial laminiert wird. Bandförmige Laminatstreifen werden aus dem Laminatband vereinzelt.

In diesem Sinne können bei einem ersten Trägermaterial insbesondere seine absorbierenden Eigenschaften wichtig sein. Bevorzugt ist die Watte eine hochreine Verbandswatte aus 100% Baumwolle. Alternativ kann als Fasermaterial ein synthetisches Material dienen. Bevorzugt ist das Fasermaterial aber ein Viskosematerial. In dieser alternativen Ausführungsform könnte die Watte eine hochreine Verbandswatte aus 100% Viskose sein.

Im Sinne der vorliegenden Erfindung können unter dem thermoplastischen Vliesstoff entsprechende nicht verwebte Materialien aus Polyethylen und/oder Polypropylen und/oder Polyester, oder einem entsprechenden Gemisch aus einem oder mehreren dieser Kunststoffe, oder einem Gemisch aus einem Polyethylen und/oder Polypropylen und/oder Polyester mit einem Fasermaterial verstanden werden. Die Materialien können so gewählt sein, dass sie sich in einem bestimmten Temperaturbereich verformen und somit eine stoffschlüssige Verbindung mit dem Trägermaterial eingehen können.

Im Sinne der vorliegenden Erfindung entsteht durch ein Laminieren ein Laminatband. Das Laminatband ist dadurch gekennzeichnet, dass die laminierten Materialien mindestens abschnittsweise, insbesondere über ihre ganze Kontaktfläche eine stoffschlüssige Verbindung eingegangen sind. Eine stoffschlüssige Verbindung kann im Sinne der vorliegenden Erfindung dann vorliegen, wenn die beiden Materialien thermoplastisch verbunden sind. Alternativ und/oder ergänzend kann eine solche auch vorliegen, wenn die Materialien miteinander verklebt sind, z.B. durch eine Adhäsionsschicht auf einem oder beiden Schichtmaterialien, oder mechanisch miteinander Faserverbunden werden, z.B. durch Verkämmen und/oder Verkarden.

In einer besonderen Ausführungsform umfasst das Zusammenführen des Trägermaterials und des Schichtmaterials ein Übereinanderlegen der beiden Schichten, sodass zumindest eine Flächenseite des Trägermaterials mit einer Flächenseite des Schichtmaterials kontaktiert ist.

In einer besonderen Ausführungsform liegen das Trägermaterial und das Schichtmaterial als Bänder vor, und das Zusammenführen umfasst einen Schritt, in dem die beiden Bänder in Längsrichtung deckungsgleich geführt werden. Dabei können Anpressrollen vorgesehen sein, welche das Zusammenführen erleichtern.

In einer besonderen Ausführungsform bezieht sich das Zusammenführen lediglich auf einen Teil der möglichen Kontaktfläche. So kann z.B. ein Band mit einem Überlappungsbereich mit dem nächsten Band zusammengeführt werden, sodass z.B. ein Flächenanteil des thermoplastischen Vliesstoffes nicht auf dem Fasermaterial aufliegt, sondern als freies Ende verbleibt. Für die Ausführung des erfindungsgemässen Verfahrens ist es von untergeordneter Bedeutung, welches Material beim Zusammenführen oben liegt und welches unten.

In einer besonderen Ausführungsform wird das Trägermaterial aber so an eine Stelle des Zusammenführens des Trägermaterials mit dem Schichtmaterial geführt, dass das Schichtmaterial von unten an das Trägermaterial geführt wird und somit das Trägermaterial auf dem Schichtmaterial aufliegt.

In einer besonderen Ausführungsform umfasst das Laminieren des Trägermaterials und des Schichtmaterials zu einem Laminatband ein Verbinden dieser beiden Bänder durch Beaufschlagung mit Energie, insbesondere von Wärme.

In einer besonderen Ausführungsform wird das Laminieren zu einem Laminatband mittels mindestens einer Laminierungsrolle durchgeführt.

In einer weiteren besonderen Ausführungsform findet das Laminieren unmittelbar nach dem Zusammenführen statt. Bevorzugt findet das Laminieren durch ein Durchführen der beiden Schichten, des Trägermaterials und des Schichtmaterials, zwischen zwei Rollen statt, wobei eine Laminierungsrolle einer Gegendruckrolle gegenüberliegend angeordnet sein kann. Alternativ zur Gegendruckrolle kann ein Gegendruckblech vorgesehen sein oder ein paar gegenüberliegender Laminierungsrollen.

In einer besonderen Ausführungsform wird der thermoplastische Vliesstoff, welches das Schichtmaterial bildet, durch Beaufschlagung mit Wärme aufgeweicht, sodass es sich mit der Zellulose des Fasermaterials verbindet.

In einer besonderen Ausführungsform ist die Laminierungsrolle eine beheizbare Rolle. Alternativ kann auch mittels eines Heissluftgebläses und/oder mittels Infrarotstrahlung genügend Wärme aufgebracht werden, um das Laminieren zu vollziehen.

Durch das Laminieren entsteht aus dem Trägermaterial und dem Schichtmaterial ein Laminatband, welches auf mindestens einem Teil seiner Oberfläche Fasermaterial aufweist und auf mindestens einem Teil seiner gegenüberliegenden Oberfläche z.B. ein thermoplastisches Vliesmaterial aufweist.

Selbstverständlich können die erfindungsgemässe Vorrichtung und das erfindungsgemässe Verfahren auch mit Laminatbändern durchgeführt werden, welche mehr als zwei unterschiedliche Schichten umfassen, oder die besagten Materialien mehrfach aufweisen. So können z.B. zwei Schichtmaterialien mit dem Fasermaterial zusammengeführt werden, sodass beide Oberflächen des resultierenden Laminatbandes zumindest z.T. mit Schichtmaterial bedeckt sind.

Durch das Laminieren eines Laminatbandes aus einem Trägermaterial und einem Schichtmaterial kann beispielsweise ein Tampon erzeugt werden, welcher eine angenehmere Haptik aufweist. Der thermoplastische Bestandteil, vorzugsweise ein Polyethylen/Polypropylen-gemisch oder ein Polyethylen/Polyester-gemisch, kann für eine verbesserte Einfuhr des Tampons im Gebrauch sorgen. Die Fasern werden besser zusammengehalten, und wenn durch den später erfolgenden Wickelprozess der thermoplastische Vliesstoff aussen zu liegen kommt, verhindert er ein Kleben der Zellulosefasern beim Kontakt mit Feuchtigkeit.

Im Sinne der vorliegenden Erfindung können sowohl das Trägermaterial als auch das Schichtmaterial als Endlosbänder verarbeitet werden. Entsprechend wird in der vorliegenden Anmeldungsbeschreibung zwischen einem Band und einem Streifen unterschieden, wobei ein Band sich auf eine undefinierte Länge bezieht, während ein Streifen in seiner Längsausdehnung begrenzt ist.

Im Sinne der vorliegenden Erfindung ist ein Materialstreifen oder ein Laminatstreifen ein in seiner Längsausdehnung begrenzter Bandbereich. Mit anderen Worten kann, zum Beispiel, ein über gewisse Längen geschnittenes Band zu einer Reihe an Laminatstreifen oder Materialstreifen führen. Für das erfindungsgemässe Verfahren können diese eine Länge aufweisen, die vom gewünschten Durchmesser, oder der gewünschten Dichte des zu erzeugenden Tampons beeinflusst ist. Vorzugsweise befinden sich die Längen in einem Bereich von zwischen 100 und 350 mm.

Im Sinne der vorliegenden Erfindung kann als Wickeleinheit eine Einheit verstanden werden, die geeignet ist, eine Wickelgabel auf einer Umlaufbahn zu fördern, und über mindestens einen Teil dieser Umlaufbahn in eine Eigenrotation zu bringen, so dass ein in einen Gabelabstand gedrückter Abschnitt eines Material- oder Laminatbandes aufgewickelt werden kann.

In einer besonderen Ausführungsform ist die Wickeleinheit als Wickeltrommel ausgebildet. Alternativ kann die Wickeleinheit auch als Wickelband ausgebildet sein.

Im Kontext der vorliegenden Erfindung kann als Wickeltrommel zum Beispiel eine Einheit mit radial angeordneten Wickelgabeln verstanden werden. Die Wickelgabeln können entlang eines Umfangs bewegt werden, zum Beispiel in einer Rotationsrichtung der Wickeltrommel, und dabei mindestens abschnittsweise in eine Rotation um ihre eigenen Längsachsen versetzt werden.

Im Kontext der vorliegenden Erfindung ist eine Rolle als eine im Wesentlichen zylindrische, kreis- oder walzenförmige Anordnung zu verstehen, welche bei einer Rotation um ihre Rotationsachse entlang ihres Umfangs eine Kurve beschreibt. Erfindungsgemässe Rollen müssen allerdings nicht zwangsläufig einen kreisrunden Querschnitt aufweisen. Entscheidend für die Funktion der Rolle ist eine im Wesentlichen umlaufende Aussenfläche, welche um einen Fixpunkt rotierbar ist. So sind einige der in der vorliegenden Erfindung als Rollen bezeichneten Bauteile auch als umlaufende Förderbänder ausgestaltbar.

In einer besonderen Ausführungsform ist die Antriebsrolle eine im Wesentlichen zylindrische Rolle, welche senkrecht zu ihrem Rotationsradius angetrieben werden kann, sodass die Rolle ein Drehmoment erhält.

Ein besonderer Vorteil der vorliegenden Erfindung liegt darin, dass die Antriebsrolle den Laminatstreifen in die Wickeltrommel schiebt. Dadurch wird kein Bauteil erforderlich, welches sich zwischen der kontinuierlich drehenden, trommelartig ausgebildeten Wickeltrommel und der Zufuhr des Laminatbandes wirken muss. Das Schieben kann durch diese besagte Antriebsrolle weiter verbessert werden, indem diese z.B. mittels einer gerippten, gerillten und/oder aufgerauten Oberfläche eine verbesserte Haftreibung bzgl. des Laminatstreifens ausgestaltet wird, um diesen Laminatstreifen somit in eine Förderrichtung zu bewegen.

Besonders bevorzugt ist die Antriebsrolle so gesteuert, dass sie mit einer bestimmten Geschwindigkeit dreht. Diese Geschwindigkeit ist bevorzugt so gewählt, dass der Laminatstreifen in die Wickeltrommel beschleunigt wird. Insbesondere soll der Laminatstreifen relativ zur Geschwindigkeit des Material- oder Laminatbandes zulaufseitig zur Antriebsrolle beschleunigt werden.

Besonders bevorzugt ist die Geschwindigkeit der Antriebsrolle so gewählt, dass die Beschleunigung des Material- oder Laminatstreifens in die Wickeltrommel ausreichend ist, um diesen zwischen dem Schwert und einem Rotationsradius der Wickelgabeln zu positionieren.

In einer besonderen Ausführungsform ist das Schwert als ein rechtwinkelig zur Längsachse der Laminatstreifen ausgebildeter Bolzen ausgestaltet, insbesondere mit einem im Wesentlichen zylindrischen Umfang. Durch den runden Umfang kann verhindert werden, dass die Material- oder Laminatstreifen sich an der Oberfläche des Schwerts aufrauen, oder materialmässig anderweitig beschädigt werden könnten.

In einer weiteren besonderen Ausführungsform hat das Schwert ein Querprofil welches ausgelegt ist ein Aufrauen Laminatstreifen sich an der Oberfläche des Schwerts im Wesentlichen zu verhindern. Dazu kann das Schwert zum Beispiel eine Querprofil aufweisen, welches im Wesentlichen Tropfenförmig oder Tragflügelförmig ist, wobei eine gerundete Oberfläche vorzugsweise in Stirnseite zur Rotationsrichtung der Wickelgabeln ausgebildet ist.

Die Wickelgabeln können im erfindungsgemässen Verfahren eine Rotation um ihre eigenen Längsachsen vollführen. Dabei rotieren sie gleichzeitig um die Rotationsachse der Wickeltrommel.

In einer besonderen Ausführungsform pausieren die Wickelgabeln beim Passieren des Schwertes die Eigenrotation. Bei der nachfolgenden Rotationsbewegung entlang des Rotationsradius der Wickeltrommel nimmt die Wickelgabel die Eigenrotation wieder auf und der Material- oder Laminatstreifen wird aufgewickelt.

In einer besonderen Ausführungsform passiert die Wickelgabel das Schwert, indem dieses mittig durch zwei Wickelgabelfinger passiert wird. Besonders bevorzugt findet dies mit pausierten Wickelgabeln statt, d.h. während die Eigenrotation der Wickelgabeln pausiert ist. Im Anschluss an das Passieren des Schwerts kann die Eigenrotation wieder aufgenommen, und der Laminatstreifen so aufgewickelt werden.

In einer besonderen Ausführungsform vollführt das Schwert eine Bewegung entgegen der Rotationsrichtung der Wickelgabel und zurück. Dies kann zum Beispiel bewerkstelligt werden, indem das Schwert ausgelegt ist einen Hub zu vollführen. Besonders bevorzugt findet der Hub synchronisiert mit der Rotationgeschwindigkeit der Wickelgabeln statt, so dass besonders bevorzugt ein vollständiger Hub in dem Zeitraum stattfindet, den die Wickelgabel benötigt, um das Schwert zu passieren. Dadurch unterstützt das Schwert ein Einführen des Laminatstreifens in die Wickelgabel und ermöglicht ein schnelleres Betreiben, indem die Rotationsgeschwindigkeit erhöht werden kann. Ein weiterer Vorteil des Hubs kann sich darin äussern, dass die Eigenrotation der Wickelgabeln über einen längeren Zeitraum erfolgen kann; so kann zum Beispiel durch früheres Einfädeln der Zeitraum in dem gewickelt werden kann verlängert werden. Insbesondere kann die Wickelgabel zum Beispiel früher die Eigenrotation wieder aufnehmen, und das Aufwickeln des Material- oder Laminatbandes einsetzen.

In einer besonderen Ausführungsform umfasst das erfindungsgemässe Verfahren einen Schwächungsschritt, bei dem das Materialband eine Sollrissstelle erhält. Dies kann zum Beispiel dadurch erfolgen, dass das Materialband einen Perforationsschritt durchläuft, bevor von der Antriebsrolle ein Materialstreifen vereinzelt wird.

In einer besonderen Ausführungsform umfasst das erfindungsgemässe Verfahren einen Perforationsschritt, bei dem das Trägermaterial perforiert wird. Bevorzugt wird das Trägermaterial vor dem Zusammenführen des Trägermaterials und des Schichtmaterials perforiert.

In einer besonders bevorzugten Ausführungsform umfasst diese Perforation des Trägermaterials eine Schwächung des Trägermaterials, indem eine Sollreissstelle geschaffen wird. Dies kann insbesondere durch speziell angepasste Rollen erreicht werden, welche mittels einer Bezahnung Ausnehmungen in das Laminatband schneiden.

In einer besonderen Ausführungsform wird auch das Schichtmaterial perforiert. Besonders bevorzugt wird das Schichtmaterial vor dem Zusammenführen des Trägermaterials und des Schichtmaterials perforiert. Analog zum Trägermaterial kann dies mit einer weiteren Stanzrolle vollbracht werden, die entsprechende Ausnehmungen als spätere Sollbruchstelle in das Trägermaterial stanzt. In dieser besonderen Ausführungsform ist zwischen dem Perforieren des Schichtmaterials und dem Zusammenführen des Trägermaterials und des Schichtmaterials eine weitere angetriebene Rolle vorgesehen, welche das Schichtmaterial bzgl. der Fördergeschwindigkeit zulaufseitig dieser weiteren Antriebsrolle beschleunigt wird. Durch diese Beschleunigung kann ein Abreissen des Schichtmaterials erfolgen.

In einer besonderen Ausführungsform ist das Laminatband zulaufseitig von der Antriebsrolle ein durchgehendes Band des Trägermaterials mit beabstandet angeordneten Perforationen, worauf bereits vereinzelte Streifen des Schichtmaterials laminiert sind.

In einer besonderen Ausführungsform erfolgt das Vereinzeln des Laminatbandes zu Laminatstreifen durch die Antriebsrolle. Dabei kann eine Drehgeschwindigkeit der Antriebsrolle so gewählt werden, dass sie das Laminatband verglichen mit der zulaufseitigen Fördergeschwindigkeit beschleunigt. Dadurch wird ein Laminatstreifen vom Laminatband abgerissen und vereinzelt. Die gewünschte Länge der Laminatstreifen kann durch die Wahl und Steuerung der Antriebsrolle von einem Fachmann, was ein weiterer Vorteil der vorliegenden Erfindung darstellt, stufenlos geregelt werden.

In einer besonderen Ausführungsform des erfindungsgemässen Verfahrens rotiert die Wickelgabel im Gegenuhrzeigersinn um die eigene Achse.

In einer anderen besonderen Ausführungsform des erfindungsgemässen Verfahrens wird zulaufseitig der Antriebsrolle ein Rückholfaden um das Material- oder Laminatband geschlagen. Bevorzugt wird dieses rechtwinkelig zur Längsachse des Material- oder Laminatbandes geschlagen.

In einer weiteren besonderen Ausführungsform wird der Material- oder Laminatstreifen durch eine Fortsetzung der Rotationsbewegung der Wickelgabel, nachdem ein Abschnitt des Material- oder Laminatstreifens in den Gabelabstand gedrückt wurde, gewickelt. Das Wickeln kann z.B. anhand einer Führungskurve erfolgen, entlang welcher die Eigenrotation der Wickelgabeln den Material- oder Laminatstreifen aufwickelt. Die Führungskurve kann insbesondere so ausgestaltet sein, dass sie den gesamten Wickelvorgang begleitet, und sie ist in ihrem Kurvenradius daher bevorzugt so zu wählen, dass sie im Wesentlichen der zu verwickelnden Streifenlänge entspricht.

Bevorzugt rotiert die Wickeltrommel um eine Rotationsachse, welche horizontal ausgerichtet ist. Die Rotation der Wickeltrommel erfolgt vorzugsweise im Uhrzeigersinn. Somit werden die Wickelgabeln im Uhrzeigersinn an das Schwert herangeführt, wobei sich die Wickelgabeln selbst im Gegenuhrzeigersinn um ihre Rotationsachse drehen, und so um das Schwert bewegen, dass dabei ein zwischen Schwert und Wickelgabel hineingefördertes Stück Laminatstreifen mitgezogen und später verwickelt wird.

In einer besonderen Ausführungsform wird die Wickelgabel an einer Schliessstation vorbeigeführt, welche den Wickel schliesst. Dies kann ein thermoplastisches Schliessen sein, analog wie es oben beschrieben wurde bzgl. des Laminats, respektive des Laminierens. Insbesondere kann dieses Schliessen erfolgen, indem ein Flächenanteil eines thermoplastischen Vliesstoffes das beim Laminieren nicht auf dem Fasermaterial aufgelegen, sondern als freies Ende verblieben ist, mit dem Wickel thermoplastisch verbunden wird. Analog und/oder ergänzend kann dies auch mit einer anderen Methode zur stoffschlüssigen Verbindung bewerkstelligt werden. Bevorzugt wird dabei die gleiche Methode angewandt, wie zuvor beim Erzeugen des entsprechenden Laminatbandes, z.B. eine Methode ausgewählt aus der Gruppe bestehend aus: Verkämmen, Verkleben und Verfilzen. Im Sinne der vorliegenden Erfindung kann durch das stoffschlüssige Verbinden des Flächenanteils, das beim Laminieren nicht auf dem Fasermaterial aufgelegen ist mit dem erzeugten Wickel ein Schliessen des Wickels bewerkstelligt werden.

In einer weiteren besonderen Ausführungsform wird die Wickelgabel an einem "Anti-Telescoping-Modul" vorbeigeführt, bei dem ein allfällig die Trägerschicht überlappender Bereich an Schichtmaterial so angeformt wird, dass ein proximaler Abschluss am fertigen Tampon erreicht ist.

Mit dem erfindungsgemässen Verfahren kann eine kontinuierliche Fertigung bewerkstelligt werden, die skalierbar ist. Durch die Ausgestaltung der Trommel ist den einzelnen Prozessschritten, wie dem Wickeln und/oder Schliessen, dennoch genug Zeit eingeräumt, um hohe Qualitäten zu erhalten.

In einer besonderen Ausführungsform wird entlang des gesamten Wickelprozesses in der Wickeltrommel eine koaxial zu den Wickelgabeln wirkende Saugkraft auf diese ausgeübt. Dadurch kann z.B. ein umgeschlagener Rückholfaden im Bearbeitungsverlauf stabilisiert werden. Dies kann zum Beispiel über Ansaugrohre bewerkstelligt werden.

Das erfindungsgemässe Verfahren kann leicht an unterschiedliche Tampongrössen angepasst werden. So kann über die Steuerung der Vereinzelungsschritte die Tampongrösse gesteuert werden. Abgestimmte Perforationen und Beschleunigungen der Antriebsrolle ermöglichen es, Bänder in der gewünschten Länge zu erhalten. Gleichzeitig kann z.B. die Führungskurve austauschbar ausgestaltet sein, sodass sie an eine entsprechende Wickellänge angepasst werden kann.

Auch die Wickelgabeln und das Schwert können in ihrem Gabelabstand und im Schwertdurchmesser ausgetauscht werden, sodass verschiedene Bandhaptiken und Dicken akkommodiert werden können.

Ein weiterer Aspekt der vorliegenden Erfindung betrifft eine Vorrichtung zum Wickeln von Vorformlingen zur Herstellung von Tampons. Bevorzugt ist die Vorrichtung zur Ausführung des oben beschriebenen Verfahrens geeignet.

Die Vorrichtung umfasst eine Fördereinrichtung zum Zuführen eines Materialbandes, insbesondere eines endlosen Materialbandes.

Sie umfasst weiter eine Vereinzelungseinheit, zum Vereinzeln von bandförmigen Materialstreifen aus dem Materialband, sowie eine Antriebsrolle zum Einschieben der Laminatstreifen in eine Wickeleinheit. In einer besonders bevorzugten Ausführungsform wirkt die Antriebsrolle die Vereinzelungseinheit, indem sie zum Beispiel mit einer Geschwindigkeit fördert, die höhere ist als die Geschwindigkeit, in welcher die Fördereinrichtung das Materialband zuführt.

In einer besonderen Ausführungsform ist das Materialband als Laminatband ausgebildet, wie oben erläutert. Dann umfasst die Vorrichtung eine erste Zugrolle zum Zuführen eines Trägermaterials, insbesondere eines Fasermaterials. Die Zugrolle kann z.B. mit Zähnen, Zacken und/oder Aufrauhungen versehen sein, welche eine Haftreibung der Rolle bzgl. des Trägermaterials erhöhen. Die Zugrolle ist angetrieben, sodass sie bei ihrer Rotation um ihre Rotationsachse das Trägermaterial von einer Trägermaterialrolle zuführt.

In einer besonderen Ausführungsform ist ein Rollenmagazin vorgesehen, welches eine Reihe von Trägermaterialrollen umfasst. Besondere Trägermaterialpuffer können beim Versiegen einer Trägermaterialrolle dazu dienen, dass nahtlos eine weitere Rolle zugeführt wird, sodass das kontinuierliche Herstellungsverfahren nicht durch einen Ersatz der Trägermaterialrollen unterbrochen werden muss. Analog kann dies auch für Schichtmaterialrollen vorgesehen sein. Die erfindungsgemässe Vorrichtung umfasst nämlich eine zweite Zugrolle zum Zuführen eines Schichtmaterials, insbesondere eines thermoplastischen Vliesstoffes. Dabei kann diese zweite Zugrolle analog wie die erste Zugrolle mit entsprechenden Förderhilfen ausgestaltet sein. Die erfindungsgemässe Vorrichtung umfasst weiterhin eine Laminierungseinheit zum Laminieren eines Laminatbandes aus dem Trägermaterial und dem Schichtmaterial.

In einer besonderen alternativen Ausführungsform umfasst das Rollenmagazin mindestens ein Trägermagazin in dem das Trägermaterial geschichtet angeordnet ist.

In einer besonderen Ausführungsform ist die Laminierungseinheit eine beheizbare Rolle. Bevorzugt wird zudem eine entsprechende Gegenrolle angeordnet, welche einen Anpressdruck auf die beheizte Rolle auszuüben vermag, sodass ein zwischen den beiden Rollen geführtes Band an Trägermaterial und Schichtmaterial zu einem Laminatband laminiert wird.

Alternativ kann das Laminieren auch über nicht beheizte Rollen stattfinden, die mittels einer Induktion, z.B. einem Infrarotstrahler, an der Anpressstelle erwärmt werden.

In einer alternativen weiteren Ausführungsform findet das Laminieren lediglich über ein Anpressen statt, indem die Materialien des Trägermaterials und des Schichtmaterials so ausgelegt sind, dass sie von sich aus eine stoffschlüssige Verbindung eingehen können. So kann z.B. das Schichtmaterial mit einer Klebstoff- und/oder Adhäsionsschicht vorgesehen sein, die an dem Trägermaterial bindet. Ebenfalls denkbar wäre es, eine Adhäsion vorgängig zu induzieren. Dies kann z.B. dadurch erfolgen, dass ein Schichtmaterial vor dem Zusammenführen erwärmt wird, z.B. über einen Infrarotstrahler, welches adhäsive Eigenschaften auf dem Schichtmaterial und/oder dem Trägermaterial aktiviert und dann beim Zusammenführen lediglich die beiden Materialien aufeinandergedrückt werden müssen.

Die erfindungsgemässe Vorrichtung umfasst weiter eine Vereinzelungseinheit zum Vereinzeln von bandförmigen Laminatstreifen aus dem Laminatband. Zudem umfasst sie mindestens eine Antriebsrolle zum Einschieben der Laminatstreifen in eine rotierbare Wickeltrommel. Mögliche funktionale Ausgestaltungen für die Antriebsrolle wurden bereits vorgängig geschildert. Die Antriebsrolle kann über einen Antriebsrollenhebel auf das Laminatband gepresst werden, sodass eine optimale Förderwirkung besteht. Der Antriebsrollenhebel kann auch einfach dazu ausgestaltet sein, die Antriebsrolle bei Bedarf vom Laminatband zu heben, wobei die Funktion des Laminatbandes unterbleibt und das Band nicht weiter in die Wickeltrommel eingeschoben wird.

In einer besonderen Ausführungsform ist der Antriebsrollenhebel ausgestaltet, einen Druckausgleich auszuführen.

Die erfindungsgemässe Vorrichtung umfasst weiter eine Wickeleinheit, die eine Mehrzahl an Wickelgabeln aufweist. Diese Wickelgabeln sind so angeordnet, dass sie an einem ortsfest oder bewegbar bzgl. der Förderung der Wickeleinheit angeordneten Schwert vorbeiführbar sind. Dies kann z.B. dadurch bewerkstelligt werden, dass das Schwert sich von einer ortsfesten Halterung für das Schwert in den Förderraum der Wickelgabeln erstreckt. Alternativ kann das Schwert bewegbar gelagert sein, zum Beispiel indem ein schwenkbarer Hebelsteg in Wirkverbindung mit einem Getriebe zur Halterung des Schwerts im Förderraum der Wickelgabeln vorgesehen ist. Besonders bevorzugt ist das Schwert ausgelegt einen Hub zu vollführen.

In einer besonderen Ausführungsform ist die Wickeleinheit als Wickeltrommel ausgebildet. Die Wickeltrommel weist eine Mehrzahl an Wickelgabeln auf. Diese Wickelgabeln sind so angeordnet, dass sie an einem ortsfest oder bewegbar bzgl. einer Rotation der Wickeltrommel angeordneten Schwert vorbeiführbar sind. Dies kann z.B. dadurch bewerkstelligt werden, dass das Schwert sich von einer ortsfesten Halterung für das Schwert in den Rotationsradius der Wickelgabeln erstreckt. Alternativ kann das Schwert bewegbar gelagert sein, zum Beispiel indem ein schwenkbarer Hebelsteg in Wirkverbindung mit einem Getriebe zur Halterung des Schwerts im Rotationsradius der Wickelgabeln vorgesehen ist. Besonders bevorzugt ist das Schwert ausgelegt einen Hub zu vollführen, insbesondere wobei der Hub eine Bewegung entgegen der Rotationsrichtung der Wickeltrommel und zurück umfasst.

Die Rotationsradien der Wickelgabeln sind so ausgelegt, dass ein Gabelabstand der Wickelgabeln das Schwert während der Rotationsbewegung der Wickeltrommel berührungslos hindurchlässt. Entsprechend sind in der erfindungsgemässen Vorrichtung die Wickelgabeln so am Schwert vorbeiführbar, dass ein in die Wickeltrommel eingeschobener Laminatstreifen durch das Schwert in einem Gabelabstand gedrückt und so von der Wickelgabel aufgenommen wird. Bevorzugt sind die Wickelgabeln in ihrer Eigenrotation pausierbar, während sie derartig am Schwertvorbeigeführt werden.

In einer besonderen Ausführungsform kann eine Laufplatte vorgesehen sein, auf welche die mindestens eine Antriebsrolle den Material- oder Laminatstreifen in die rotierbare Wickeltrommel einschiebt. Diese Laufplatte kann mit einer Ausnehmung versehen sein, welche ein Passieren der Wickelgabeln der Laufplatte ermöglicht.

In einer besonderen Ausführungsform ist diese Ausnehmung der Laufplatte in der Grösse so gewählt, dass sie dem durch die Wickelgabel an das Schwert gelegte Fläche des Material- oder Laminatstreifens entspricht.

In einer besonderen Ausführungsform ist die mindestens eine Antriebsrolle zugleich die Vereinzelungseinheit zur Abtrennung von Material- oder Laminatstreifen vom Material- oder Laminatband. Beispiele, wie dies ausgeführt werden kann, sind vorgängig bereits im Zusammenhang mit dem erfindungsgemässen Verfahren beschrieben.

In einer besonderen Ausführungsform umfasst die erfindungsgemässe Vorrichtung eine Führungskurve, entlang welcher die Wickelgabeln in ihrer Bewegung entlang des Rotationsradius der Wickeltrommel den Material- oder Laminatstreifen aufwickeln können. Bevorzugt ist diese Führungskurve austauschbar ausgestaltet, sodass sie mit ihrem Kurvenradius und der Kurvenlänge an die Dimensionen eines Material- oder Laminatstreifens angepasst werden kann.

In einer besonderen Ausführungsform sind die Wickelgabeln so ausgestaltet, dass sie um ihre eigene Längsachse rotierbar sind, d.h. in eine Eigenrotation versetzt werden können. Bevorzugt können die Wickelgabeln dazu angetrieben sein, besonders bevorzugt einzeln antreibbar sein.

Dazu können z.B. Zahnräder vorgesehen sein, welche eine Rotationsbewegung der Wickeltrommeln über ein Zahnradsystem auf eine Rotation der Wickelgabeln übertragen. Bevorzugt sind die einzelnen Wickelgabeln so ausgestaltet, dass sie einzeln angetrieben zu rotieren vermögen. Dadurch kann zum Beispiel ein Pausieren der Eigenrotation der Wickelgabeln auf das Passieren des Schwertes abgestimmt werden, und die Eigenrotation anschliessend beim Wickelvorgang wieder aufgenommen werden.

In einer besonderen Ausführungsform umfasst die Vorrichtung weiter mindestens je eine Führungsrolle zur Stabilisation des Trägermaterials, des Schichtmaterials und des Laminatbandes. Um ständig eine gute Band- und Materialbandspannung sicherzustellen, können Führungsrollen vorgesehen sein, welche einerseits ein laterales, d.h. ein rechtwinkelig zur Längsausdehnung des Bandes Verschieben zu verhindern, und andererseits für eine nötige Bandspannung sorgen, indem sie über Federn eine bestimmte Zugkraft auf das Band ausüben.

In einer besonderen Ausführungsform umfasst die Wickeltrommel eine weitere Schliessstation. Diese weitere Schliessstation kann dazu dienen, einen Wickel zu schliessen und zu stabilisieren.

In einer weiteren besonderen Ausführungsform umfasst die Wickeltrommel ein Anti-Telescoping-Modul, wie eingangs geschildert.

Bevorzugt ist das Schwert ausgestaltet, um eine anschlagsfreie Hub-Bewegung zu vollführen. In einer besonders bevorzugten Ausführungsform ist ein Exzenter zur Vollführung des Hubs vorgesehen. Dadurch kann das bewegbare Schwert mit einer hohen Frequenz synchron zum jeweiligen Passieren einer Wickelgabel den Hub vollführen.

Für einen Fachmann ergibt es sich von selbst, dass sämtliche sich nicht ausschliessende Ausführungsformen in einer beliebigen Kombination erfindungsgemässen Ausgestaltung verwirklicht sein können, so zum Beispiel auch durch Vorrichtungsmerkmale, die sich aus Verfahrensmerkmalen ergeben und umgekehrt.

Mit dem erfindungsgemässen Verfahren und der zugehörigen Vorrichtung ist es möglich, eine kontinuierliche Bearbeitung von Material- und/oder Laminatbändern zu Vorformlingen zu gewährleisten. Die Prozesse sind dabei skalierbar und können in einem Endlosbetrieb stattfinden. Weitere Vorteile der erfindungsgemässen Vorrichtung wurden bereits eingangs geschildert oder im Zusammenhang mit besonderen Ausführungsformen beschrieben. Für einen Fachmann versteht es sich von selbst, dass in einer erfindungsgemässen Ausgestaltung eines Verfahrens zur Herstellung von Vorformlingen von Tampons, sowie einer erfindungsgemässen Vorrichtung die geschilderten Merkmale in einer beliebigen Kombination verwirklicht sein können, sofern sie sich nicht gegenseitig ausschliessen.

Im Folgenden wird die Erfindung nun anhand konkreter Ausführungsbeispiele und Figuren näher erläutert, ohne auf diese beschränkt zu sein. Der Einfachheit halber sind in den Figuren die gleichen Elemente mit den gleichen Bezugszeichen versehen. Die Figuren legen die erfinderischen Konzepte schematisch dar und erheben keinen Anspruch auf Massstabstreue.

### Figurenbeschrieb

Anhand der nachfolgenden Figuren werden Ausführungsbeispiele der Erfindung beschrieben.

Es zeigen:
- Fig. 1: schematisch eine Übersicht der erfindungsgemässen Vorrichtung;
- Fig. 2: schematisch einen Übergabebereich in eine Wickeltrommel;
- Fig. 3a: schematisch die Annäherung einer Wickelgabel an einen Laminatstreifen;
- Fig. 3b: das Kontaktieren des Laminatstreifens durch die Wickelgabel;
- Fig. 3c: das Umbiegen eines Teils des Laminatstreifens um die Wickelgabel;

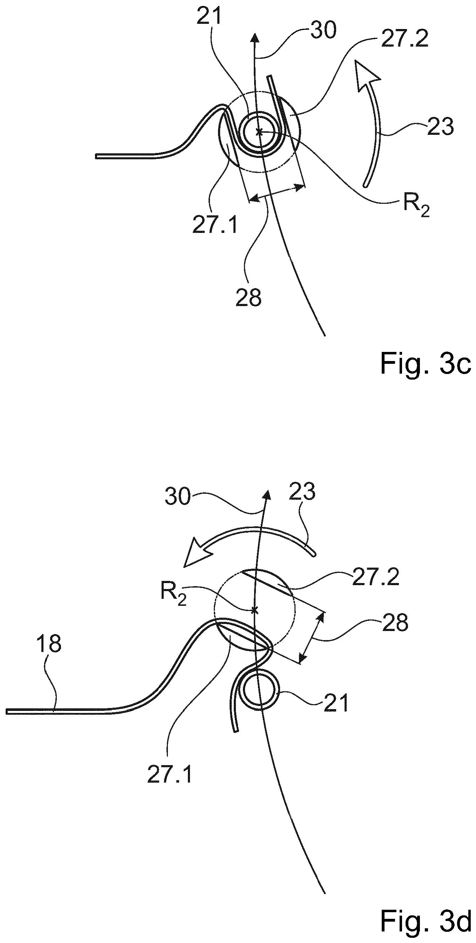

- Fig. 3c: die Fortsetzung entlang des Rotationsradius der Wickeltrommel;
- Fig. 3d: schematisch den Beginn des Wickelprozesses durch die Wickelgabel;

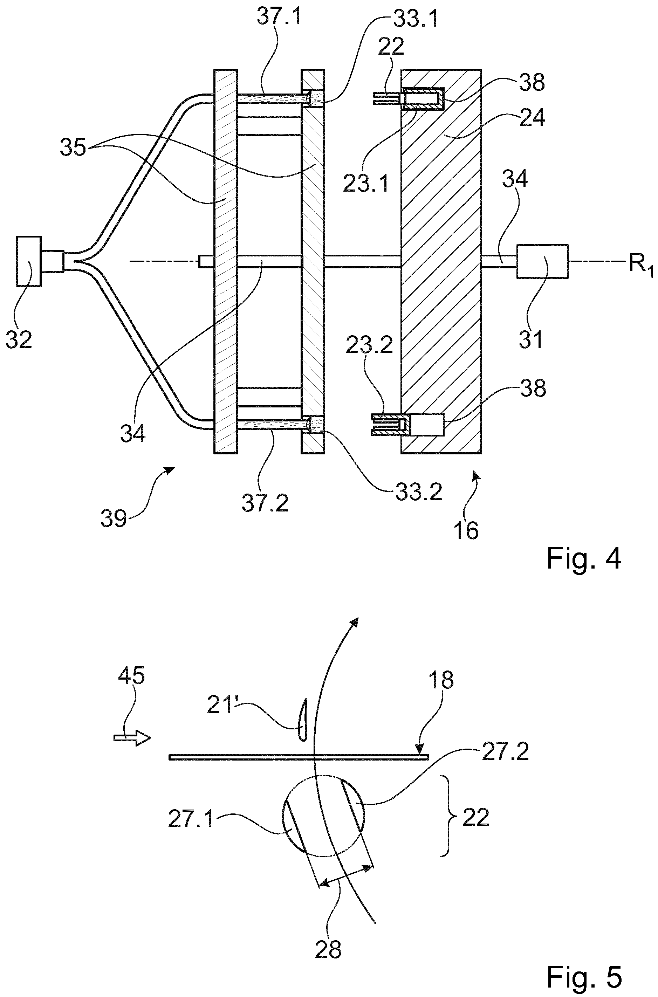

- Fig. 4: schematisch einen Querschnitt durch die Wickeltrommel und die Ausstosstrommel;
- Fig. 5: eine alternative Ausgestaltung eines Schwerts, und

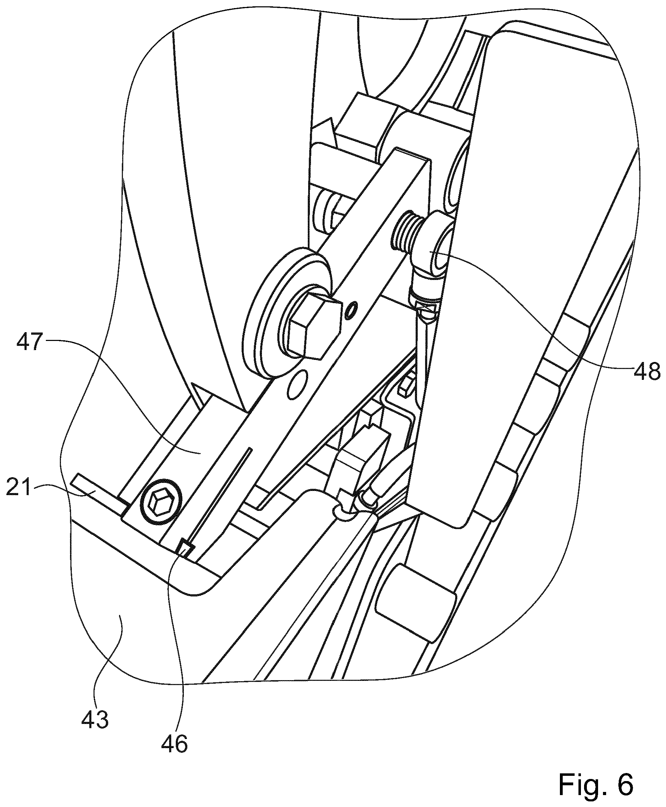

- Fig. 6: eine alternative Ausgestaltung als bewegbares Schwert.

### Ausführung der Erfindung

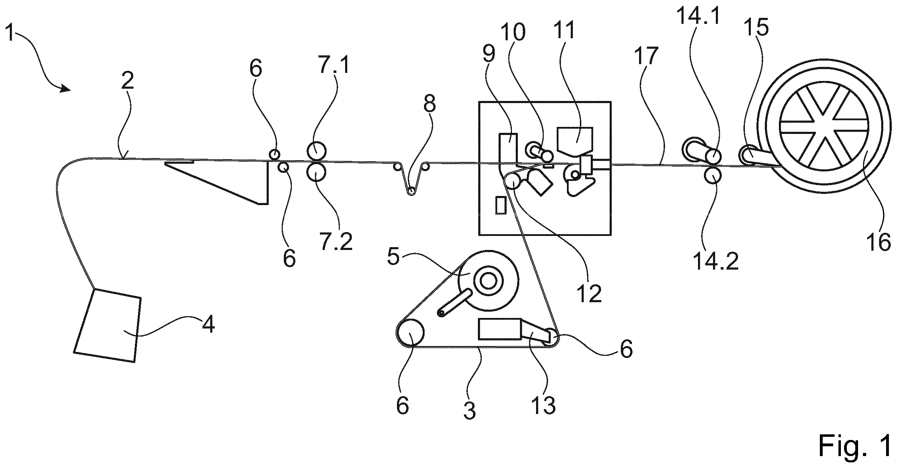

Die **Fig. 1** zeigt schematisch, wie eine erfindungsgemässe Vorrichtung aufgebaut sein kann. In dieser schematischen Ansicht findet eine Förderbewegung von links nach rechts statt. Dabei werden zwei bandförmige Materialien zusammengeführt, verschweisst und als Laminatband einer Wickeltrommel 16 zugeführt. Das Trägermaterial 2 stammt dazu von einer Trägermaterialrolle 4, von welcher das Trägermaterial 2 durch einen kontinuierlichen Zug zugeführt wird. Der Zug auf die rotierbar gelagerte Trägermaterialrolle 4 stammt von einer ersten Zugrolle 7.1, welche auf das Trägermaterialband 2 eine Zugkraft durch die Rotation um ihre Rotationsachse ausübt. Im vorliegenden Ausführungsbeispiel ist der ersten Zugrolle 7.1 eine Gegendruckrolle 7.2 gegenübergestellt, zwischen welche das Band des Trägermaterials 2 geführt ist. Die Gegendruckrolle 7.2 kann auch angetrieben werden, wodurch in einem besonderen Beispiel zwei erste Zugrollen 7.1, 7.2 für den notwendigen Zug auf das Band verantwortlich wären. Im vorliegenden Beispiel ist das Trägermaterial 2 ein Watteband aus Baumwollzellulosefasern. Das Trägermaterial 2 bildet im späteren Tampon das hauptsächliche saugfähige Material für die Aufnahme von Flüssigkeit.

Für besondere Anwendungen, z.B. gewisse hämostatische oder medizinale Tampons, ist eine Quellwirkung von unerwünschter oder untergeordneter Bedeutung. In diesen Beispielen ist das Trägermaterial 2 entsprechend anders gewählt. Bei medizinischen Tampons, die für die Abgabe eines Wirkstoffs ausgelegt sind, kann das Trägermaterial zu diesem Zeitpunkt bereits mit dem entsprechenden Wirkstoff versehen sein. Es ist auch möglich, den Wirkstoff später auf das fertige Tamponprodukt zu applizieren. Entlang der gesamten Förderrichtung bis hin zur Wickeleinrichtung 16 können eine Reihe von Führungsrollen 6 vorgesehen sein, welche die Bandführung stabilisieren und ein seitliches Verschieben des entsprechenden Bandes unterbinden. Im vorliegenden Beispiel ist zulaufseitig von der ersten Zugrolle ein Paar an Führungsrollen 6 ausgebildet, welche eine im Wesentlichen horizontale Zufuhr der Bandbreite zur ersten Zugrolle 7.1 gewährleisten.

Neben Führungsrollen 6 kann eine Bandspannung mittels entlang der Bandstrecke angebrachter Ausgleichsrollen 8 gewährleistet werden. Eine adäquate Bandspannung garantiert, dass der Wirkeingriff der Antriebs- und Zugrollen das entsprechende Band optimal fördert, sowie die auf das Band einwirkenden Werkzeuge stets auf einer im Wesentlichen glatten Bandoberfläche arbeiten können. Im vorliegenden Beispiel ist die Ausgleichsrolle 8 nach der ersten Zugrolle 7.1 in Bandlaufrichtung angeordnet. Dabei kann die Ausgleichsrolle 8 mittels einer Feder, eines Aktuators oder eines anderen Bandausgleichsmittels ausgestattet sein, die eine entsprechende Rückstellkraft auf das Band auszuüben vermögen.

Im vorliegenden konkreten Beispiel ist die Ausgleichsrolle 8 gefedert ausgestaltet, sodass sie auf das durch sie verspannte Band eine Rückstellkraft, welche proportional zur Federkraft ausübt. Das Trägermaterial 2 wird weiter zu einer Perforationseinheit 9 befördert. Diese Perforationseinheit 9 dient im vorliegenden Beispiel dazu, Schwächungen in das Trägermaterial 2 zu stanzen.

Dazu kann die Perforationseinheit 9 z.B. als bezahnte Walze ausgestattet sein, die entlang ihres Umlaufradius einen oder mehrere Schneidzähne aufweist, welche bei der Abrollbewegung über das Trägermaterial 2 dieses entsprechend perforieren. Alternativ kann auch ein rechtwinkelig zur Bandlaufrichtung verlaufendes und alternierend hin und her schwenkendes Schneidmesser vorgesehen sein, um die nötige Schwächung des Trägermaterials 2 zu ermöglichen. Während der kontinuierlichen Zufuhr des Trägermaterials 2 zur Perforationseinheit 9 wird von einer Schichtmaterialrolle 5 gleichzeitig und ebenso kontinuierlich ein Schichtmaterial 3 abgehaspelt. Alternativ kann auch ein Schichtmaterialmagazin vorgesehen sein, in dem das Schichtmaterial geschichtet, z.B. ziehharmonikaartig gefaltet aufbewahrt ist.

Beim Schichtmaterial 3 handelt es sich im vorliegenden Beispiel um ein thermoplastisches Vlies, welches ein Polyethylen/Polypropylen-gemisch aufweist. Unter Umständen muss auf die Materialien durch die Anordnung besonderer Führungsrollen Rücksicht genommen werden. Im vorliegenden Beispiel kann z.B. eine Anordnung von weiteren Führungsrollen 6 für die Zufuhr des Schichtmaterials 3 der vom Watteband des Trägermaterials 2 abweichenden Elastizität des thermoplastischen Vlieses des Schichtmaterials 3 Rechnung getragen werden.

Im vorliegenden konkreten Ausführungsbeispiel ist direkt nach der Schichtmaterialrolle eine erste Führungsrolle 6 angeordnet und anschliessend ein Bandspannungshebel 13 mit einer angeschlossenen Führungsrolle 6 vorgesehen. Dieser Bandspannungshebel kann federnd schwenkbar gelagert sein, sodass er eine entsprechende Bandspannung aufrechtzuerhalten hilft. Eine zweite Zugrolle ist dafür vorgesehen, das Schichtmaterial 3 von der Schichtmaterialrolle 5 abzuhaspeln und in Richtung des Trägermaterials 2 zu fördern. Grundsätzlich sind dem Fachmann mögliche Gestaltungsweisen von solchen Zugrollen 7.1, 12 bekannt. In einem konkreten Beispiel können die Zugrollen 7.1, 12 Rollenumfänge aufweisen, die eine entsprechende Oberflächenstruktur haben, um den Förderprozess zu erleichtern. Besonders bewährt hat sich dabei eine Profilierung der Rollenoberfläche. So können z.B. Rillen, Zähne oder Aufrauhungen auf der Rollenoberfläche den Förderprozess erleichtern. Solche Ausgestaltungsweisen sind dem Fachmann bekannt.

Das Schichtmaterial 3 gelangt in der vorliegenden Ansicht von unten an das Trägermaterial 2. Dies ist jedoch keine Voraussetzung. Es ist ebenso denkbar, dass das Schichtmaterial 3 von oben auf das Trägermaterial 2 geführt wird, wobei geringfügige Anpassungen auch im weiteren Prozessverlauf vorgenommen werden müssen. Über eine Anpressrolle 10 werden die beiden Bänder 2, 3 miteinander physisch kontaktiert, sodass eine Oberfläche des Trägermaterials 2 mindestens z.T. von dem Schichtmaterial 3 unterlegt ist. Im Anschluss an die Anpressrolle 10, welche neben der Mitnahme des Schichtmaterialbands durch das Trägermaterial 2 auch eine Schneidvorrichtung vorsehen kann für das Schichtmaterial 3 vorsehen kann, gelangen die kontaktierten Bänder 2, 3 zur Laminierungseinheit 11.

In einer anderen Ausführungsform findet das Zuschneiden des Schichtmaterials direkt nach der zweiten Zugrolle 12 in Bandlaufrichtung statt (nicht gezeigt). Die Laminierungseinheit 11 ist im vorliegenden Beispiel dazu ausgestaltet, eine entsprechende Wärme auf die beiden Bänder auszuüben, sodass diese durch die thermoplastische Aufweichung des Schichtmaterials 3 stoffschlüssig verbunden werden.

Ein nunmehr entstandenes Laminatband 17 hat zwei Schichten, nämlich eine Watte- und eine thermoplastische Vliesschicht. Das Laminatband 17 wird über eine Förderrolle 14.1 und ihre entsprechende Gegenrolle 14.2 zu einer Antriebsrolle (in dieser Figur nicht gezeigt) geführt, welche über einen Antriebsrollenhebel 15 mit der nötigen Kraft auf das Laminatband 17 gedrückt wird und indem ihre Rotationsgeschwindigkeit schneller ist als die der Förderrolle 14.1 eine Beschleunigung eines Laminatbandteils erzeugt, welches einen Laminatstreifen von diesem durch Reissen der Sollbruchstelle oder Schwächung ermöglicht. Dieser Laminatstreifen wird in der Wickeltrommel 16 weiterverarbeitet.

Der gesamte Prozessverlauf der vorliegenden Vorrichtung kann kontinuierlich erfolgen. So können z.B. magazinhaft ausgestaltete Rollen einen nahtlosen Übergang ermöglichen und eine kontinuierliche Bandzufuhr der Trägermaterialien 2 und Schichtmaterialien 3 ermöglichen.

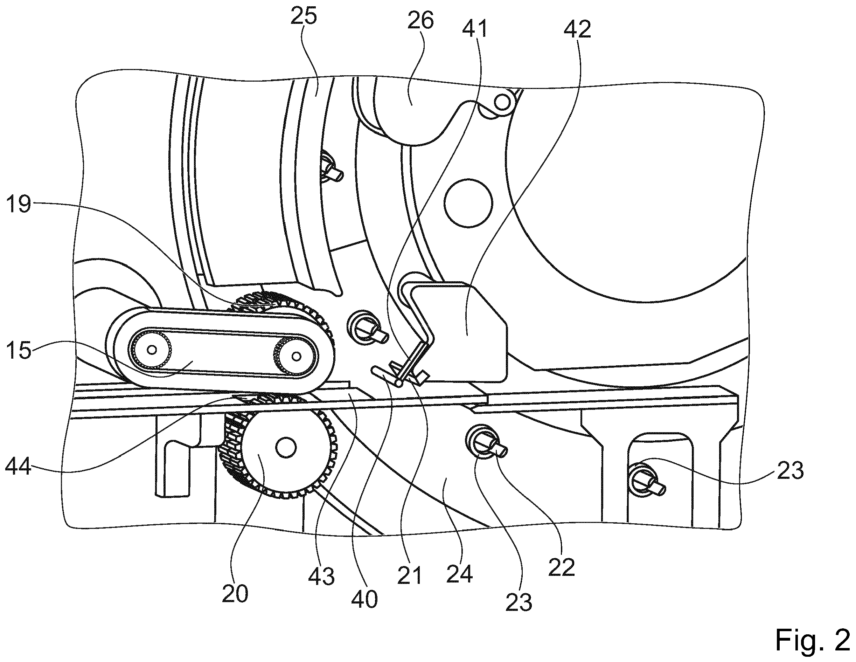

Die Fig. 2 zeigt schematisch den Übertrittspunkt der Laminatstreifen in die Wickeltrommel, wobei zur besseren Darstellung das Innenleben der Wickeltrommel 16 dargestellt ist. Wie vorgängig erwähnt, gelangt das Laminatband durch die Förderrolle zur Antriebsrolle 19. Die Antriebsrolle 19 ist mittels des Antriebsrollenhebels so gelagert, dass sie in Wirkverbindung mit dem Laminatband gebracht werden kann. Entsteht eine Wirkverbindung, wird durch ihre relative höhere Drehgeschwindigkeit um ihre Rotationsachse ein Stück des Laminatbandes abgetrennt und in die Wickeltrommel als Laminatstreifen befördert. Im vorliegenden Beispiel wird die Antriebsrolle 19 dabei durch eine ebenfalls angetriebene Gegendruckrolle 20 unterstützt.

Das Laminatband wird bei der Einfuhr in die Wickeltrommel 16 auf einer Laufplatte 43 geführt, welche eine Laufplattenausnehmung 44 aufweist, durch welche die Gegendruckrolle 20 mit dem Laminatband in Wirkverbindung treten kann. Mit dieser Rollenanordnung wird der Laminatstreifen praktisch in die Wickeltrommel geschoben, ohne dass ein physisch mechanischer Hebel oder eine Übertragungseinheit das Laminatband schrittweise in die Wickeltrommel befördern muss. Dadurch kann die Wickeltrommel 16 ebenfalls kontinuierlich operiert werden, und höhere Prozessgeschwindigkeiten sind möglich. Beim Einschub des Laminatstreifens in die Wickeltrommel 16 gelangt der Laminatstreifen mit mindestens einem Teil seiner Fläche zwischen ein Schwert und einen Rotationsradius, der radial entlang des Umfangs der Wickeltrommel 16 angeordneten Wickelgabeln 22.

Die in diesem Trommelumfang rotierenden Wickelgabeln 22 treten durch ihre Bewegung mit dem Laminatstreifen in Wirkverbindung und führen diesen mittels einer Eigenrotation um das Schwert 21 herum. Dabei drückt das Schwert 21 einen Teil des Laminatstreifens in einen Wickelgabelabstand der Wickelgabeln 22, wodurch diese in der Lage sind, den Laminatstreifen mitzubefördern. In der Wickeltrommel ist ebenfalls eine auswechselbare Führungskurve 25 aus Kunststoff vorgesehen, welche als Gegenfläche für die Wicklung der Laminatstreifen um die Wickelgabeln dient. Die um ihre eigene Achse rotierenden Wickelgabeln 22 sind auf einer Drehscheibe 24 angeordnet. Weiter kann die Wickeltrommel 16 mit zusätzlichen Bearbeitungseinheiten ausgestattet sein. Im vorliegenden Beispiel ist z.B. eine Schliessstation vorgesehen, welche die fertigen Wickel mittels thermischer stoffschlüssiger Verbindung des thermoplastischen Materials analog, wie dies beim Schichtmaterial stattgefunden hat, verschliesst.

Das Schwert 21 ist so angeordnet, dass es in den Rotationsradius der Wickelgabeln auf der Wickeltrommel ragt. Dazu ist im vorliegenden Beispiel eine Halterung für das Schwert 42 vorgesehen, die mittels eines Stegs 41 das Schwert 21 senkrecht zur Längsrichtung der Laminatstreifen und parallel zur Rotationsachse der Wickeltrommel hält. Die Wickelgabeln 22 können über einen eigenen Antrieb für ihre Eigenrotation verfügen und sind von Hülsen 23 umgeben.

Alternativ kann das Schwert 21 auch bewegbar angeordnet sein, indem zum Beispiel der Steg aktuierbar ausgestaltet ist, zum Beispiel zum Vollzug eines Hubs, so dass das Schwert insgesamt eine Bewegung entgegen der Rotationsrichtung der Wickelgabel 22 und zurückzuvollführen vermag.

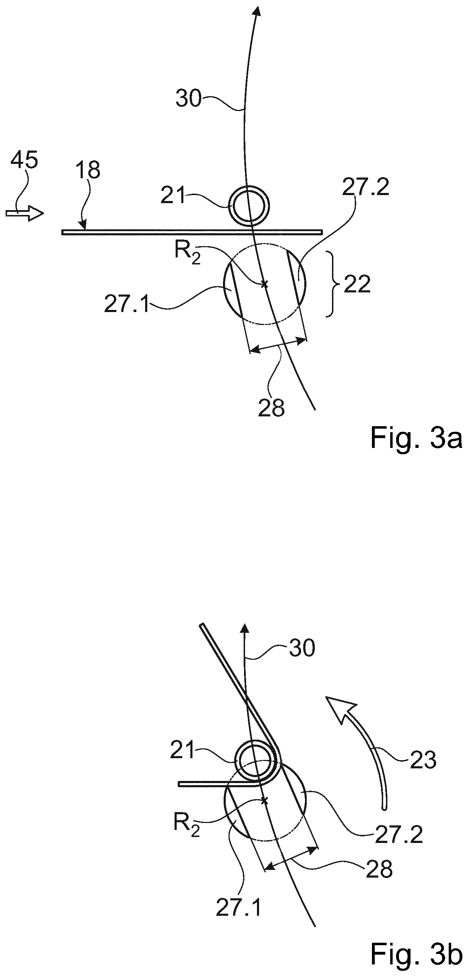

Um die Mitnahme der Laminatstreifen 18 durch die Wickelgabeln 22 zu veranschaulichen, ist der Prozess in den Fig. 3a bis 3d nochmals schematisch und vereinfacht dargestellt. Dabei sollen diese Figuren lediglich die einzelnen Schritte veranschaulichen, ohne Anspruch darauf zu erheben, die genaue Ausrichtung der Wickelgabeln zu einem bestimmten Prozesszeitpunkt genau darzulegen.

Die Fig. 3a zeigt, wie der Laminatstreifen 18 in den Wirkbereich des Schwerts 21 und der sich entlang eines Rotationsradius 30 der Wickeltrommel bewegenden Wickelgabel 22 gelangt. Die Wickelgabel 22 umfasst im vorliegenden Beispiel zwei Wickelgabelfinger 27.1, 27.2, welche einen dazwischenliegenden Gabelabstand 28 definieren. Eine Rotationsachse R2 der Wickelgabel 22 bewegt sich entlang des Rotationsradius 30 der Wickeltrommel. Im Betrieb ist die Wickelgabel 22 so ausgestattet, dass sie um ihre Rotationsachse R2 rotiert. Obschon im vorliegenden Beispiel eine Wickelgabel 22 mit zwei Wickelgabelfingern 27.1, 27.2 dargestellt ist, sind selbstverständlich auch Ausführungsformen möglich mit mehr als zwei Wickelgabelfingern. So ist ein System mit drei Fingern ebenso denkbar wie eine Anordnung mit zahlreichen einzelnen Dornen als Finger. Das Schwert 21 ist im Verlauf des Rotationsradius 30 so angeordnet, dass bei der Rotation der Wickelgabeln 22 um den Rotationsradius 30 bei einer Eigenrotation um die Rotationsachse R2 diese das Schwert 21 kontaktlos passieren. Dazu kann die Wickelgabel 22 in ihrer Eigenrotation für den Zeitraum pausieren, den die Wickelgabel benötigt, um das Schwert zu passieren.

In der Fig. 3b ist eine Wickelgabel 22 in ihrer Eigenrotation pausiert und bereits so in Kontakt mit einem Teil des Laminatstreifens 18 getreten, dass ein Wickelgabelfinger 27.2 einen Teil des Laminatstreifens um das Schwert umbiegt. Die Rotation der Wickelgabel 22 um ihre Rotationsachse R2 im Gegenuhrzeigersinn 23 ist pausiert.

In der Fig. 3c sind die Wickelgabelfinger 27.1, 27.2 auf gleicher Höhe wie das Schwert. Die Wickelgabeln 22 sind im Verhältnis zum Schwert 21 so ausgestaltet, dass der ein Gabelabstand 28 ausreicht, um das Schwert berührungslos zu passieren. Während die Wickelgabel 22 das Schwert 21 kontaktlos passiert, indem die beiden Wickelgabelfinger 27.1, 27.2 auf der Rotations-Umlaufbahn um das Schwert 21 geführt werden, wird ein Teil des Laminatstreifens 18 vom Schwert 21 zwischen die beiden Wickelgabelfingern 27.1 und 27.2 gedrückt. In dieser Position ist die Rotation der Wickelgabel 22 um ihre Rotationsachse R2 im Gegenuhrzeigersinn 23 pausiert.

In der Figure 3d hat die Wickelgabel 22 das Schwert 21 vollständig passiert. Bei der Weiterbewegung der Wickelgabel 22 wird der Laminatstreifen 18 weitergezogen. Die nun erneut im Gegenuhrzeigersinn 23 um ihre Rotationsachse R2 rotierende Wickelgabel 22 wickelt nun den Laminatstreifen um die Wickelgabelfinger 27.1, 27.2 auf und zieht es am Schwert 21 vorbei. Der Laminatstreifen bleibt mit einer Schlaufe in mindestens einem Wickelgabelfinger 27,1 hängen. Dadurch wird der Laminatstreifen in der folgenden Weiterbewegung vollständig aufgewickelt.

In der Fig. 4 wird eine weitere vorteilhafte Ausgestaltung der vorliegenden Erfindung gezeigt. Die Fig. 4 zeigt schematisch einen Querschnitt durch eine Wickeltrommel 16 und eine mit ihr assoziierten Saugtrommel 39. Die Wickeltrommel 16 ist rotierbar um eine Rotationsachse R1 über einen Trommelantrieb 31 durch eine Trommelachse 34 gelagert. Die Wickeltrommel 16 verfügt entlang ihres Umfangs über radial angeordnete Wickelgabeln 22. Diese Wickelgabeln 22 sind so angeordnet, dass sie in einen Zwischenraum zwischen der Wickeltrommel und der Saugtrommel 39 ragen. Die Wickelgabeln 22 sind dabei auf einer Drehscheibe 24 angeordnet. In entsprechenden Vertiefungen der Drehscheibe 24 sind rund um die Wickelgabel 22 Hülsen 23.1, 23.2 angeordnet. Diese Hülsen dienen im vorliegenden Beispiel dem Ausstoss des fertigen Wickels von der Wickelgabel, z.B. an eine Übernahmestation. Im vorliegenden Beispiel ist / sind eine Wickelgabel 23.1 in einem eingefahrenen Zustand gezeigt und eine zweite Wickelgabel 23.2 in einem ausgefahrenen Zustand. Stülpt sich die ausgefahrene Hülse 23.2 um die Wickelgabel 22, so wird ein auf der Wickelgabel 22 geformter Wickel abgestossen.

Vom gleichen Trommelantrieb 31 angetrieben über die zentrale Trommelachse 34 ist eine ebenfalls parallel zur Wickeltrommel 16 rotierende Saugtrommel 39 positioniert. Die Saugtrommel 39 umfasst zwei parallel angeordnete Drehscheiben 35. Der dem Zwischenraum zwischen der Saugtrommel 39 und der Wickeltrommel 16 zugewandten Drehscheibe 35 sind Ansaugöffnungen 33.1, 33.2 vorgesehen. Durch diese kann ein Luftstrom mittels eines Gebläses 32 eine Ansaugwirkung über Saugrohre 37.1, 37.2 erfolgen. Im Betrieb sind die Laminatstreifen resp. die Wickel oder bereits Vorformlinge mit Rückholfäden im Verlauf der Bandzufuhr versehen worden. Damit diese Rückholfäden im Prozessbetrieb sicher aus dem Weg gehalten werden können, sind die Saugrohre so ausgestattet, dass ein konstanter Sog auf die Wickel ausgeübt wird. Die Wickel ragen daher im Wesentlichen parallel zur Rotationsachse R1 der Wickeltrommel während des ganzen Prozessverlaufs in den Zwischenraum zwischen der Wickeltrommel 16 und der Saugtrommel 39. Dadurch ist sichergestellt, dass die Rückholfäden nicht mit irgendwelchen beweglichen Teilen eine ungewünschte Interaktion eingehen.

Die **Fig.** 5 zeigt eine alternative Ausgestaltung eines Schwerts 21', welches einen Profilquerschnitt aufweist, der eine Stirnseite mit einer im Wesentlichen runden Oberfläche hat. Insgesamt ist dieses alternative Schwert 21' tragflügelförmig ausgerichtet. Die gezeigte Position entspricht der Position der Fig. 3a, wo die Wickelgabel 22 in den Wirkbereich des Schwerts 21' tritt und die Wickelgabelfinger 27.1, 27.2 in einer Position die Eigenrotation der Wickelgabel pausieren, so dass ein kontaktfreies Passieren des Schwerts erfolgt. Ein in Einschubrichtung der Antriebsrolle (nicht gezeigt) eingeschobener Laminatstreifen 18 befindet sich zwischen Wickelgabel 22 und Schwert 21' und wird anschliessend umgebogen, und als Schlaufe um einen Wickelgabelfinger 27.1 gelegt, so dass bei einer Wiederaufnahme der Eigenrotation der Wickelgabel 22, dieser aufgewickelt wir. Das Querschnittprofil des alternativen Schwert 21' ist ausgestaltet, um möglichst keine scharfe Kante und/oder Reibungsstelle zum Laminatstreifen 18 zu bilden, wenn dieser von der Wickelgabel 22 weggezogen wird.

In der Figur 6 wird eine beispielhafte Ausführungsform mit bewegbarem Schwert 21 schematisch dargestellt. Diese Anordnung könnte analog in der Figur 2 die Bauteile 21, 42 und 41 ersetzen.

Das Schwert 21 ist kraft- und formschlüssig über einen keilförmig ausgebildeten Schwertstift 46 mit einem Hebelsteg 47 verbunden. Der Hebelsteg 27 weist eine zu diesem Zweck komplementäre Nut zum Schwertstift 46 aus. Durch eine Feststellschraube und einen Klemmschlitz wird das Schwert fixiert. Insgesamt ist das Schwert somit austauschbar ausgestaltet. Dies hat den Vorteil, dass mittels Auswahl das Schwert an verschiedene Kaliber von Wickelgabeln, mit verschiedenen Wickelgabelabständen angepasst werden kann.

Der Hebelsteg 47 ist über ein Gelenk 48 exzentrisch antreibbar, so dass er einen Hub vollführt. Dieser Hub äussert sich in ein Hin- und Zurückbewegen des Schwerts, wobei das Hin- und Zurück die Rotationsrichtung der Wickelgabeln sein kann. Somit lässt sich anschlagsfrei ein Hub vollführen, die mit der Rotationsgeschwindigkeit der Wickelgabeln synchronisierbar ist. Mit der vorliegenden Erfindung werden ein Verfahren und eine Vorrichtung zur Herstellung von Vorformlingen, also gewickelten Laminatstreifen, gezeigt, die kontinuierlich betreibbar ist und eine hohe Prozessqualität ermöglicht. Durch die erfindungsgemässe Vorrichtung können insbesondere sehr hohe Prozessgeschwindigkeiten möglich werden. Durch den Verzicht auf intermittierende Übertragung der Laminatstreifen wird auch der energieintensive Stop-and-go-Betrieb von Maschinenteilen so weit reduziert, dass der Verschleiss der Einzelteile reduziert wird und die Wartungsintervalle verkürzt werden.

### Bezugszeichenliste

- 1: Vorrichtung
- 2: Trägermaterial
- 3: Schichtmaterial
- 4: Trägermaterial-Rolle
- 5: Schichtmaterial-Rolle
- 6: Führungsrolle
- 7.1: erste Zugrolle
- 7.2: Gegendruckrolle zu erster Zugrolle
- 8: Ausgleichsrolle
- 9: Perforierungseinheit
- 10: Anpressrolle
- 11: Laminierungseinheit
- 12: zweite Zugrolle
- 13: Bandspannungshebel
- 14.1: Förderrolle
- 14.2: Gegendruckrolle zu Förderrolle
- 15: Antriebsrollenhebel
- 16: Wickeltrommel
- 17: Laminatband
- 18: Laminatstreifen
- 19: Antriebsrolle
- 20: Gegendruckrolle zur Antriebsrolle
- 21: Schwert
- 21': alternatives Schwert
- 22: Wickelgabel
- 23: Hülse
- 24: Drehscheibe
- 25: Führungsblech
- 26: Schliessstation
- 27: Wickelgabelfinger
- 28: Gabelabstand
- 29: Drehsinn der Wickelgabel
- 30: Drehsinn der Wickeltrommel
- 31: Trommelantrieb
- 32: Gebläse
- 33.1: Saugöffnung
- 33.2: Saugöffnung
- 34: Trommelachse
- 35: Drehscheiben der Saugtrommel
- 36: Verstrebung
- 37.1: Saugrohr
- 37.2: Saugrohr
- 38: Hülsenaufnahme
- 39: Saugtrommel
- 40: Lenkblech
- 41: Steg für Schwert
- 42: Halterung für Schwert
- 43: Laufplatte
- 44: Ausnehmung der Laufplatte
- 45: Einschubrichtung der Antriebsrolle
- 46: Schwertstift
- 47: Hebelsteg
- 48: Gelenk

- R1: Rotationsachse der Wickeltrommel
- R2: Rotationsachse der Wickelgabel

## Patentansprüche

1. Verfahren zum Wickeln von Vorformlingen zur Herstellung von Tampons, umfassend die Schritte:
a. Zuführen eines Materialbands, insbesondere eines endlosen Materialbands;
b. Vereinzeln von bandförmigen Materialstreifen aus dem Materialband, und
**dadurch gekennzeichnet, dass**
ein **Materialstreifen** jeweils mittels mindestens einer **Antriebsrolle** (19) in eine kontinuierlich fördernde **Wickeleinheit** dergestalt eingeschoben wird, dass der Materialstreifen zwischen eine **Wickelgabel** (22) und einem **Schwert** (21) so positioniert wird, dass bei **einer Bewegung** der Wickelgabel (22) um das Schwert (21), dieses einen Abschnitt des Materialstreifens (18) in einen **Gabelabstand (28)** drückt.

2. Verfahren gemäss Anspruch 1, wobei das Materialband als Laminatband (17) ausgebildet ist, weiter umfassend die Schritte:
a. Zuführen eines Trägermaterials (2), insbesondere eines Fasermaterials, mittels mindestens einer ersten Zugrolle (7.1, 7.2);
b. Zuführen eines Schichtmaterials (3), insbesondere eines thermoplastischen Vliesstoffes, mittels mindestens einer zweiten Zugrolle (12);
c. Zusammenführen des Trägermaterials (2) und des Schichtmaterials (3);
d. Laminieren eines Laminatbands (17) aus dem Trägermaterial (2) und dem Schichtmaterial (3), und wobei der Schritt des Vereinzelns von bandförmigen Materialstreifen aus dem Materialband ein Vereinzeln von bandförmigen Laminatstreifen (18) aus dem Laminatband (17) ist.

3. Verfahren gemäss einem der Ansprüche 1 oder 2, wobei die fördernde Wickeleinheit eine rotierende Wickeltrommel (16) ist auf der die Wickelgabeln radial angeordnet sind.

4. Verfahren gemäss einem der Ansprüche 2 oder 3, wobei das Trägermaterial (2) perforiert wird, insbesondere wobei das Trägermaterial vor dem Schritt c) perforiert wird.

5. Verfahren gemäss einem der Ansprüche 2 bis 4, wobei das Schichtmaterial (3) perforiert wird, insbesondere wobei das Schichtmaterial (3) vor dem Schritt c) perforiert wird.

6. Verfahren gemäss einem der Ansprüche 1 bis 5, wobei das Vereinzeln durch die Antriebsrolle (19) erfolgt, insbesondere indem eine Antriebsgeschwindigkeit der Antriebsrolle (19) höher ist als eine Fördergeschwindigkeit des Materialbands zulaufseitig der Antriebsrolle (19).

7. Verfahren gemäss einem der Ansprüche 1 bis 6, wobei die Wickelgabel das Schwert passiert, so dass der Materialstreifen, insbesondere der Laminatstreifen (18), zwischen Wickelgabel (22) und Schwert (21) eingefädelt wird.

8. Verfahren gemäss einem der Ansprüche 1 bis 7, wobei zulaufseitig der Antriebsrolle ein Rückholfaden um das Materialband, insbesondere um das Laminatband (17) geschlagen wird, insbesondere rechtwinklig zur Längsachse des Materialbands, insbesondere des Laminatbands (17) geschlagen wird.

9. Verfahren gemäss einem der Ansprüche 1 bis 8, wobei der Materialstreifen, insbesondere Laminatstreifen (18) durch eine Rotationsbewegung der Wickelgabel (22), nachdem ein Abschnitt des Materialstreifens, insbesondere des Laminatstreifens (18) in den Gabelabstand (28) gedrückt wurde, gewickelt wird.

10. Verfahren gemäss Anspruch 9, wobei der gewickelte Materialstreifen oder Laminatstreifen (18) von **einer Schliessstation (26)** verschlossen wird.

11. Verfahren gemäss einem der Ansprüche 1 bis 10, wobei das Schwert eine Bewegung entgegen der Rotationsrichtung der Wickelgabel und zurück vollführt.

12. **Vorrichtung** (1) zum Wickeln von Vorformlingen zur Herstellung von Tampons, insbesondere zur Durchführung eines Verfahrens gemäss Anspruch 1, umfassend:
eine **Fördereinrichtung** zum Zuführen eines Materialbands, insbesondere eines endlosen Materialbands
eine **Vereinzelungseinheit,** zum Vereinzeln von bandförmigen Materialstreifen (18) aus einem Materialband (17),
mindestens eine **Antriebsrolle (19)** zum Einschieben der Laminatstreifen (18) in **eine Wickeleinheit,**
die **Wickeleinheit** (16) umfassend **eine Mehrzahl an Wickelgabeln (22),** welche so angeordnet sind, dass sie an einem ortsfest oder bewegbar bezüglich der Bewegung der Wickelgabeln angeordneten **Schwert** (21) dergestalt vorbeiführbar sind, dass ein in die Wickeleinheit (16) eingeschobener Laminatstreifen (18) durch das Schwert (21) in **einen Gabelabstand** (28) der Wickelgabeln gedrückt, und so von der Wickelgabel (22) aufgenommen wird.

13. Vorrichtung gemäss Anspruch 12, wobei das Materialband als Laminatband (17) ausgebildet ist, die Fördereinrichtung umfassend:
a. Eine **erste Zugrolle** (7.1, 7.2) zum Zuführen eines Trägermaterials (2), insbesondere eines Fasermaterials;
b. Eine **zweite Zugrolle** (12) zum Zuführen eines Schichtmaterials (3), insbesondere eines thermoplastischen Vliesstoffes;
c. Eine **Laminierungseinheit** (11) zum Laminieren eines Laminatbands (17) aus dem Trägermaterial (2) und dem Schichtmaterial (3).

14. Vorrichtung gemäss einem der Ansprüche 12 oder 13, wobei die Wickeleinheit als **Wickeltrommel** (16) ausgebildet ist, insbesondere wobei die Wickeleinheit als kontinuierlich drehbare, trommelartig ausgebildeten Wickeltrommel (16) mit radial angeordneten Wickelgabeln (22) ausgebildet ist.

15. Vorrichtung gemäss einem der Ansprüche 12 bis 14, wobei die mindestens eine Antriebsrolle (19) die Vereinzelungseinheit ist.

16. Vorrichtung gemäss einem der Ansprüche 14 oder 15, weiter umfassend **eine Führungskurve (25)** entlang welcher die Wickelgabel (22) in ihrer Bewegung entlang des Rotationsradius der Wickeltrommel (16) den Laminatstreifen (18) aufwickelt.

17. Vorrichtung gemäss einem der Ansprüche 14 bis 16, wobei die Wickelgabeln (22) um ihre eigene Längsachse rotierbar auf der Wickeltrommel angeordnet sind.

18. Vorrichtung gemäss einem der Ansprüche 13 bis 17, wobei die Vorrichtung weiter mindestens je eine Führungsrolle (6) zur Stabilisation des Trägermaterials (2), des Schichtmaterials (3) und des Laminatbands (17) umfasst.

19. Vorrichtung gemäss einem der Ansprüche 12 bis 18, wobei das Schwert (21) ortsfest im Rotationsradius der Wickelgabeln (22) auf der Wickeltrommel angeordnet ist, so dass das Schwert durch den Gabelabstand (28) der Wickelgabeln kontaktfrei von diesen auf ihrem Rotationsradius passierbar ist.

20. Vorrichtung gemäss einem der Ansprüche 12 bis 18, wobei das Schwert bewegbar im Rotationsradius der Wickelgabeln (22) auf der Wickeltrommel angeordnet ist, so dass das Schwert durch den Gabelabstand (28) der Wickelgabeln kontaktfrei von diesen auf ihrem Rotationsradius passierbar ist, insbesondere wobei das Schwert ausgestaltet ist einen Hub zu vollführen.

21. Vorrichtung gemäss einem der Ansprüche 11 bis 17, wobei die Wickeltrommel eine **Schliessstation** (26) umfasst.

## Claims

1. A method for winding preforms for manufacturing tampons, comprising the steps:
a. feeding a material tape, in particular an endless material tape;
b. separating tape-shaped material strips from the material tape, and
**characterized in that**
a material strip is pushed into a continuously conveying winding unit by means of at least one drive roller (19) in such a way that the material strip is positioned between a winding fork (22) and a guide bar (21) in such a way that when the winding fork (22) moves around the guide bar (21), the latter pushes a section of the material strip (18) into a fork gap (28).

2. The method of claim 1, wherein the material tape is designed as a laminate tape (17), further comprising the steps:
a. feeding a backing material (2), in particular a fiber material, by means of at least one first draw roller (7.1, 7.2);
b. feeding a layered material (3), in particular a thermoplastic non-woven fabric, by means of at least one second draw roller (12);
c. bringing together the backing material (2) and the layered material (3);
d. laminating a laminate tape (17) from the backing material (2) and the layered material (3), and wherein the step of separating tape-shaped material strips from the material tape consists in separating strip-shaped laminate strips (18) from the laminate tape (17).

3. The method of any one of claims 1 or 2, in which the conveying winding unit is a rotating winding drum (16) on which the winding forks are arranged radially.

4. The method of any one of claims 2 or 3, in which the backing material (2) is perforated, in particular in which the backing material is perforated before step c).

5. The method of any one of claims 2 to 4, in which the layered material (3) is perforated, in particular in which the layered material (3) is perforated before step c).

6. The method of any one of claims 1 to 5, wherein the separation is performed by the drive roller (19), in particular in that a drive speed of the drive roller (19) is higher than a conveying speed of the material tape on the upstream side of the drive roller (19).

7. The method of any one of claims 1 to 6, wherein the winding fork passes the guide bar so that the material strip, in particular the laminate strip (18), is threaded between the winding fork (22) and guide bar (21).

8. The method of any one of claims 1 to 7, in which a removal thread is wrapped around the material tape, in particular the laminate tape (17), on the upstream side of the drive roller, in particular perpendicular to the longitudinal axis of the material tape, in particular the laminate tape (17).

9. The method of any one of claims 1 to 8, wherein the material strip, in particular laminate strip (18) is wound by a rotational movement of the winding fork (22) after a section of the material strip, in particular the laminate strip (18) has been pressed into the fork gap (28).

10. The method of claim 9, wherein the wound material strip or laminate strip (18) is closed by a closing station (26).

11. The method of any one of claims 1 to 10, wherein the guide bar performs a movement counter to the direction of rotation of the winding fork and back.

12. A device (1) for winding preforms for manufacturing tampons, in particular for carrying out a method according to claim 1, comprising:
a conveyor device for feeding in a material tape, in particular an endless material tape
a separating unit for separating tape-shaped material strips (18) from a material tape (17),
at least one drive roller (19) for pushing the laminate strips (18) into a winding unit,
the winding unit (16) comprising a plurality of winding forks (22) which are arranged in such a way that they can be guided past a guide bar (21) which is stationary or movable with respect to the movement of the winding forks in such a way that a laminate strip (18) pushed into the winding unit (16) is pressed by the guide bar (21) into a fork gap (28) of the winding forks, and is thus received by the winding fork (22).

13. The device according to claim 12, wherein the material tape is designed as a laminate tape (17), the conveyor device comprising:
a. a first draw roller (7.1, 7.2) for feeding a backing material (2), in particular a fiber material;
b. a second draw roller (12) for feeding a layered material (3), in particular a thermoplastic non-woven fabric;
c. a laminating unit (11) for laminating a laminate tape (17) from the backing material (2) and the layered material (3).

14. The device of any one of claims 12 or 13, wherein the winding unit is designed as a winding drum (16), in particular wherein the winding unit is designed as a continuously rotatable, drum-like winding drum (16) with radially arranged winding forks (22).

15. The device of any one of claims 12 to 14, wherein the at least one drive roller (19) is the separating unit.

16. The device of any one of claims 14 or 15, further comprising a guide curve (25) along which the winding fork (22) winds up the laminate strip (18) in its movement along the rotational radius of the winding drum (16).

17. The device of any one of claims 14 to 16, wherein the winding forks (22) are arranged on the winding drum so as to be rotatable about their own longitudinal axis.

18. The device of any one of claims 13 to 17, wherein the device further comprises at least one respective guide roller (6) for stabilizing the backing material (2), the layered material (3) and the laminate tape (17), respectively.

19. The device of any one of claims 12 to 18, wherein the guide bar (21) is arranged stationary in the rotation radius of the winding forks (22) on the winding drum, so that the guide bar can pass through the fork gap (28) of the winding forks without contacting them on their rotation radius.

20. The device of any one of claims 12 to 18, wherein the guide bar is arranged stationary in the rotation radius of the winding forks (22) on the winding drum, so that the guide bar can pass through the fork gap (28) of the winding forks without contacting them on their rotation radius, in particular wherein the guide bar is designed to perform a stroke.

21. The device of any one of claims 11 to 17, wherein the winding drum comprises a closing station (26).

## Revendications

1. Procédé d'enroulement de préformes pour la fabrication de tampons, comprenant les étapes de :
a. alimentation d'une bande de matériau, en particulier d'une bande de matériau sans fin ;
b. séparation de rubans de matériau de type bande à partir de la bande de matériau, et
**caractérisé en ce que**
un ruban de matériau est respectivement inséré au moyen d'au moins un rouleau d'entraînement (19) dans une unité d'enroulement à transport continu, que le ruban de matériau est positionné entre une fourche d'enroulement (22) et une lame (21) de telle manière que, lors d'un mouvement de la fourche d'enroulement (22) autour de la lame (21), celle-ci presse une section du ruban de matériau (18) dans un espacement de fourche (28).

2. Procédé selon la revendication 1, dans lequel la bande de matériau est réalisée sous la forme d'une bande stratifiée (17), comprenant en outre les étapes de :
a. alimentation d'un matériau de support (2), en particulier d'un matériau fibreux, au moyen d'au moins un premier rouleau cannelé (7.1, 7.2) ;
b. alimentation d'un matériau de revêtement (3), en particulier d'un non-tissé thermoplastique, au moyen d'au moins un second rouleau cannelé (12) ;
c. combinaison du matériau de support (2) et du matériau de revêtement (3) ;
d. laminage d'une bande stratifiée (17) à partir du matériau de support (2) et du matériau de revêtement (3), et l'étape de séparation de rubans de matériau de type bande à partir de la bande de matériau consistant à séparer des rubans (18) stratifié de type bande à partir de la bande stratifiée (17).

3. Procédé selon l'une des revendications 1 ou 2, dans lequel l'unité d'enroulement de transport est un tambour d'enroulement (16) rotatif sur lequel les fourches d'enroulement sont disposées radialement.

4. Procédé selon l'une des revendications 2 ou 3, dans lequel le matériau de support (2) est perforé, en particulier dans lequel le matériau de support est perforé avant l'étape c).

5. Procédé selon l'une des revendications 2 à 4, dans lequel le matériau de revêtement (3) est perforé, en particulier dans lequel le matériau de revêtement (3) est perforé avant l'étape c).

6. Procédé selon l'une des revendications 1 à 5, dans lequel la séparation est effectuée par le rouleau d'entraînement (19), en particulier en ce que la vitesse d'entraînement du rouleau d'entraînement (19) est supérieure à la vitesse de transport de la bande de matériau côté alimentation du rouleau d'entraînement (19).

7. Procédé selon l'une des revendications 1 à 6, dans lequel la fourche d'enroulement passe devant la lame de sorte que le ruban de matériau, en particulier le ruban stratifié (18), est inséré entre la fourche d'enroulement (22) et la lame (21).

8. Procédé selon l'une des revendications 1 à 7, dans lequel, côté alimentation du rouleau d'entraînement, un fil de retour est enroulé autour de la bande de matériau, en particulier autour de la bande stratifiée (17), en particulier perpendiculairement à l'axe longitudinal de la bande de matériau, en particulier de la bande stratifiée (17).

9. Procédé selon l'une des revendications 1 à 8, dans lequel le ruban de matériau, en particulier le ruban stratifié (18), est enroulé par un mouvement de rotation de la fourche d'enroulement (22) après qu'une section du ruban de matériau, en particulier du ruban stratifié (18), a été pressé dans l'espacement de fourche (28).

10. Procédé selon la revendication 9, dans lequel le ruban de matériau ou le ruban stratifié (18) enroulé est fermé par un poste de fermeture (26).

11. Procédé selon l'une des revendications 1 à 10, dans lequel la lame effectue un mouvement dans le sens opposé au sens de rotation de la fourche d'enroulement, puis revient.

12. Dispositif (1) destiné à enrouler des préformes pour la fabrication de tampons, en particulier pour la mise en œuvre d'un procédé selon la revendication 1, comprenant :
un dispositif de transport destiné à alimenter une bande de matériau, en particulier une bande de matériau sans fin,
une unité de séparation destinée à séparer des rubans de matériau (18) de type bande à partir d'une bande de matériau (17),
au moins un rouleau d'entraînement (19) destiné à insérer les rubans stratifiés (18) dans une unité d'enroulement,
l'unité d'enroulement (16) comprenant une pluralité de fourches d'enroulement (22) qui sont disposées de sorte qu'elles peuvent passer devant une lame (21) fixe ou mobile par rapport au mouvement des fourches d'enroulement (21) de sorte qu'un ruban stratifié (18) inséré dans l'unité d'enroulement (16) est pressé par la lame (21) dans un espacement de fourche (28), et est ainsi reçu par la fourche d'enroulement (22).

13. Dispositif selon la revendication 12, dans lequel la bande de matériau est réalisée sous la forme d'une bande stratifiée (17), le dispositif de transport comprenant :
a. un premier rouleau cannelé (7.1, 7.2) destiné à alimenter un matériau de support (2), en particulier un matériau fibreux ;
b. un second rouleau cannelé (12) destiné à alimenter un matériau de revêtement (3), en particulier un non-tissé thermoplastique ;
c. une unité de laminage (11) destinée à stratifier une bande stratifiée (17) composée du matériau de support (2) et du matériau de revêtement (3).

14. Dispositif selon l'une des revendications 12 ou 13, dans lequel l'unité d'enroulement est réalisée sous la forme d'un tambour d'enroulement (16), en particulier dans lequel l'unité d'enroulement est réalisée sous la forme d'un tambour d'enroulement (16) rotatif en continu, de type tambour, muni de fourches d'enroulement (22) disposées radialement.

15. Dispositif selon l'une des revendications 12 à 14, dans lequel l'au moins un rouleau d'entraînement (19) est l'unité de séparation.

16. Dispositif selon l'une des revendications 14 ou 15, comprenant en outre une courbe de guidage (25) le long de laquelle la fourche d'enroulement (22) enroule le ruban stratifié (18) lors de son mouvement le long du rayon de rotation du tambour d'enroulement (16).

17. Dispositif selon l'une des revendications 14 à 16, dans lequel les fourches d'enroulement (22) sont disposées sur le tambour d'enroulement de manière rotative autour de leur propre axe longitudinal.

18. Dispositif selon l'une des revendications 13 à 17, le dispositif comprenant en outre au moins un rouleau de guidage (6) destiné à stabiliser respectivement le matériau de support (2), le matériau de revêtement (3) et la bande stratifiée (17).

19. Dispositif selon l'une des revendications 12 à 18, dans lequel la lame (21) est disposée sur le tambour d'enroulement de manière fixe dans le rayon de rotation des fourches d'enroulement (22), de sorte que la lame peut passer à travers l'espacement de fourche (28) des fourches d'enroulement sans contact avec celles-ci sur leur rayon de rotation.

20. Dispositif selon l'une des revendications 12 à 18, dans lequel la lame est disposée sur le tambour d'enroulement de manière mobile dans le rayon de rotation des fourches d'enroulement (22), de sorte que la lame peut passer à travers l'espacement de fourche (28) des fourches d'enroulement sans contact avec celles-ci sur leur rayon de rotation, en particulier dans lequel la lame est conçue pour effectuer une course.

21. Dispositif selon l'une des revendications 11 à 17, dans lequel le tambour d'enroulement comprend un poste de fermeture (26).
